(19) Europäisches Patentamt
European Patent Office
Office européen des brevets

(11) Publication number: 0 246 077 B1

# (12) EUROPEAN PATENT SPECIFICATION

(45) Date of publication of the patent specification: 22.11.90

(51) Int. Cl.⁵: **C07D 209/58**, C07D 307/77, C07D 333/50, A61K 31/40

(21) Application number: 87304231.1

(22) Date of filing: 13.05.87

(54) **Heteropolycyclic aromatic compounds.**

(30) Priority: 14.05.86 GB 8611762

(43) Date of publication of application: 19.11.87 Bulletin 87/47

(45) Publication of the grant of the patent: 22.11.90 Bulletin 90/47

(84) Designated Contracting States: AT BE CH DE ES FR GB GR IT LI LU NL SE

(56) References cited:
EP-A- 0 182 608
AT-B- 190 508
GB-A- 2 099 812

(73) Proprietor: THE WELLCOME FOUNDATION LIMITED, Unicorn House 160 Euston Road, London NW1 2BP(GB)

(72) Inventor: Bair, Kenneth Walter, 342, Wesley Drive, Chapel Hill North Carolina 27514(US)

(74) Representative: Rollins, Anthony John et al, Group Patents & Agreements The Wellcome Research Laboratories Langley Court, Beckenham Kent BR3 3BS(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 246 077 B1

ACTORUM AG

## Description

The present invention relates to heteropolycyclic alkanol derivatives which have been found to have biocidal activity. More specifically the invention concerns aminoalkanol derivatives containing a heteropolycyclic ring system, methods for the synthesis thereof, pharmaceutical formulations thereof and the use thereof as biocidal agents, particularly antitumor agents.

Two analogues of nitracrine (1-nitro-9-((3-dimethylaminopropyl)amino) acridine) containing 2-amino-2-methyl-1,3-propanediol and tris(hydroxymethyl)methylamine groups have been reported to have antitumor activity in some murine screening systems (Arzneim. Forsch./Drug Res. 32II, 1013 (1982)).

EP-A 0 182 608 published after the priority date of the present invention reports a novel class of heteropolycyclic aromatic alkanol derivatives which have biocidal activity. We have now discovered a preferred class of novel heteropolycyclic aromatic alkanol derivatives which have biocidal activity.

Accordingly, in a first aspect, the present invention provides a compound of the formula (I)

$ArCH_2NHR^1$ (I)

or a monomethyl or monoethyl ether thereof, the compound of formula (I) including the said ethers containing no more than 29 carbon atoms in total; an ester thereof; a salt thereof;
wherein Ar is a ring system of formula

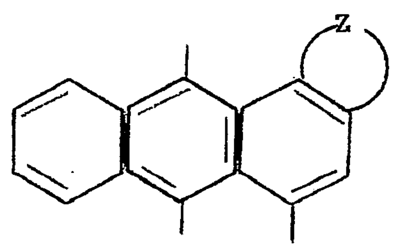

Wherein the $CH_2NHR^1$ side chain is attached to one of the indicated positions,
wherein

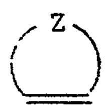

is a five-membered ring containing two double bonds, and Z is oxygen, sulphur or nitrogen substituted by hydrogen, methyl or ethyl or a resonance form thereof. Ar being optionally substituted by one or two substituents, said substituents containing not more than four carbon atoms in total when taken together and being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0,1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five-or six-membered heterocyclic ring optionally containing one or two additional heteroatoms;
$R^1$ contains not more than eight carbon atoms and is a group

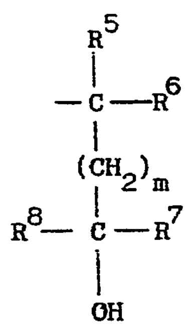

or

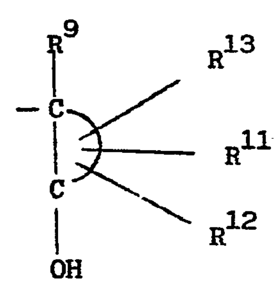

wherein m is 0 or 1;
$R^5$ and $R^6$ are the same or different and each is hydrogen or $C_{1-3}$ alkyl optionally substituted by hydroxy;
$R^7$ and $R^8$ are the same or different and each is hydrogen or $C_{1-3}$ alkyl;

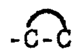 is a five-or-six-membered saturated carbocyclic ring;

$R^9$ is hydrogen, methyl or hydroxymethyl;

$R^{10}$,$R^{11}$ and $R^{12}$ are the same or different and each is hydrogen or methyl;

$R^{13}$ is hydrogen, methyl, hydroxy, or hydroxymethyl;

A preferred group of compounds of the formula (I) is that in which Ar is:

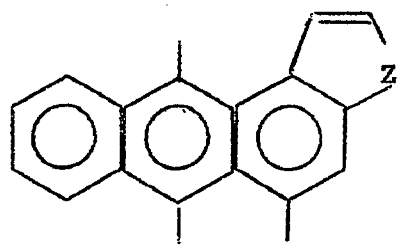

wherein Z = S, O, NMe or NEt

Suitably the aromatic ring is unsubstituted or has only one substituent. Preferably the aromatic ring is unsubstituted.

Suitably $ArCH_2NHR^1$ or a monomethyl or monoethyl ether thereof contains not more than 28 carbon atoms in total.

Suitably m is O.

Suitably $R^1$ is

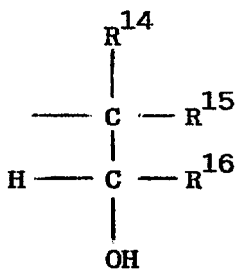

or

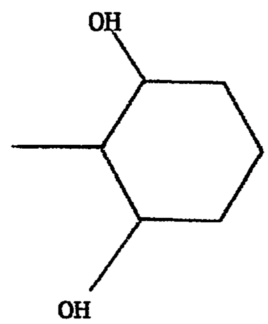

wherein $R^{14}$ is $CH_2OH$, $CH(CH_3)OH$ or $CH_2CH_2OH$;

$R^{15}$ is hydrogen, $C_{1-3}$ alkyl or $CH_2OH$;

$R^{16}$ is hydrogen or methyl.

Suitably $R^{14}$ is $CH_2OH$ or $CH(CH_3)OH$. Suitably $R^{15}$ is hydrogen,methyl, ethyl or $CH_2OH$.

Preferably $R^1$ is

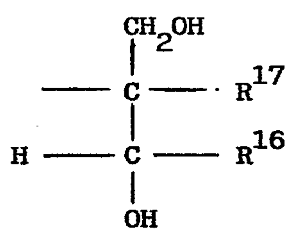

wherein $R^{16}$ is hydrogen or methyl and $R^{17}$ is hydrogen, methyl or ethyl, most preferably methyl.

Specific compounds within the scope of formula (I) include;

2-Methyl-2-[[(3-methyl-3H-naphth[2,3-e]indol-11-yl)methyl]amino]-1,3-propanediol

Salts included within the scope of the present invention are those of compounds of formula (I) and ethers and esters thereof.

Esters and non-pharmaceutically useful salts of the compounds of the formula (I) are useful intermediates in the preparation and purification of compounds of the formula (I) and pharmaceutically useful salts thereof, and are therefore within the scope of the present invention. Thus, salts of the compounds of the formula (I) useful in the present invention include those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pan-

tothenic, methanesulfonic, ethanesulfonic, benzenesulfonic, p-toluenesulfonic, naphthalene-2-sulfonic, and ascorbic acids such as glycine. Pharmacologically and pharmaceutically acceptable salts are preferred, particularly those that are soluble in solvents suitable for parenteral administration, for example, hydrochlorides, methanesulfonates and isethionates.

Esters of compounds of formula (I) are derived from acids known to those skilled in the art to be suitable for ester formation, and are conveniently those derived from $C_{1-6}$ alkanoic acids or alkanoic acid derivatives, for example acetic acid, propionic acid, n-butyric acid and iso-butyric acid. The esters may be formed from all or only some of the hydroxy groups contained in the compounds of formula (I).

The compounds of formula (I) and their ethers, esters, and salts thereof may be prepared by any method known in the art for the preparation of compounds of analogous structure. Thus, the compounds of formula (I) may, for example, be prepared by any of the methods defined below.

1. The reduction of a compound $ArCH=NR^1$ (II)
wherein Ar and $R^1$ are as hereinbefore defined, or an appropriately protected derivative thereof followed by deprotection where appropriate.

The conditions and reagents for such a reaction are well known to those skilled in the art, and any such conditions/reagents may be employed that will not reduce the aromatic ring system. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, Advanced Organic Chemistry, 2nd ed., pages 819-820, McGraw Hill, New York, 1977. The reduction is suitably carried out with $Ar\text{-}CH=NR^1$ in solution in an inert solvent or mixture of solvents compatible with the reducing agent, at a non-extreme temperature, for example, between 0° and 80°C, conveniently at room temperature.

In the case of lithium aluminum hydride and like reagents, suitable solvents include ethers (for example tetrahydrofuran, diethyl ether and 1,2-dimethoxy-ethane) optionally in the presence of a hydrocarbon cosolvent (for example toluene, benzene or hexane).

In the case of sodium borohydride and like reagents, suitably solvents include alcohols (for example ethanol, methanol or isopropanol) optionally in the presence of a hydrocarbon cosolvent (for example toluene, benzene or hexane) or an ether cosolvent (for example diethylether or tetrahydrofuran).

In the case of sodium cyanoborohydride and like reagents, suitable solvents include those described for sodium borohydride and the reduction is conveniently carried out in the presence of an acid, conveniently glacial acetic acid or ethanolic hydrochloric acid as outlined in, for example, R. Hutchins et al., Organic Preparations and Procedures International 11, 201 (1979).

In the case of catalytic hydrogenation, suitable solvents include alcohols (for example methanol and ethanol) optionally in the presence of a hydrocarbon cosolvent (for example toluene or benzene), or ether cosolvent (for example diethyl ether or tetrahydrofuran) optionally in the presence of an acid (for example glacial acetic acid or ethanolic hydrochloric acid), or glacial acetic acid.

Protected derivatives of compounds $ArCH=NR^1$ are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.

It is often convenient not to isolate the compound $ArCH=NR^1$ but to react a compound ArCHO with a compound $NH_2R^1$; wherein Ar and $R^1$ are as defined in (I) and to reduce the compound of the formula $ArCH=NR^1$ so formed in situ. The reaction of the compounds ArCHO and $NH_2R^1$ is again suitably carried out using conditions and reagents which are well known to those skilled in the art, for example in the presence of an acid, such as a sulfonic acid, i.e. p-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol. Alternatively, $ArCH=NR^1$ formed under equilibrium conditions in appropriate solvents can be reduced in situ with an appropriate reducing agent, suitably sodium cyanoborohydride.

The compound ArCHO may be in the form of a protected aldehyde, for example an acetal, which liberates the aldehyde function under the reaction conditions. In turn, a compound ArCHO can be synthesized by reacting the appropriate polycyclic aromatic hydrocarbon with a formylating agent such as that generated by the reaction between $SnCl_4$ and $Cl_2CHOCH_3$ or equivalent reagents, for example, according to the method of A. Reiche et al., Chem. Ber. 93, 88 (1960), or with other standard formylating reagents/procedures known to the art, for example, the Gatterman-Koch reaction ($CO/HCl/AlCl_3/CuCl$), the Gatterman reaction ($HCN/HCl/ZnCl_2$), and the Vilsmeier reaction ($POCl_3$/PhN(Me)CHO or $POCl_3/Me_2NCHO$) (J. March, vide supra pages 494-497). The polycyclic aromatic hydrocarbons are either known compounds or are prepared by routes analogous to those used to prepare structurally related compounds.

The compounds ArCHO may also be prepared from an appropriate aromatic heteropolycycle substituted by a suitable functional group and converting this functional group to an aldehyde group by methods well known to those skilled in the art. Suitably functional groups include $CHBr_2$, $CH_3$, $COR^{19}$ wherein $R^{19}$

is a primary or secondary $C_{1-6}$ alkyl group, COOH or a derivative thereof such as an ester, amide, or acid chloride, or CN.

Where the aromatic heteropolycycle bears substituents, ArCHO may be prepared by a variety of methods known in the art of organic chemistry depending on the nature of the substituent on the ring. For example, if the substituent(s) is a halogen, the starting materials may be prepared by direct treatment of the aromatic heteropolycycle with a halogenating agent (e.g. $Cl_2$, $Br_2$, or $SO_2Cl_2$) or indirectly by such routes as the Sandmeyer reaction (H.H. Hodgson, Chem. Rev. 40, 251 (1947). If the substituents(s) is alkyl, the aromatic heteropolycycle may be reacted with the appropriate reagents under Friedel-Crafts reaction conditions (G.A. Olah, FriedelCrafts and Related Reactions, Vols. 1-3 Interscience, New York, NY, 1963-1965).

The compounds of the formula $NH_2R^1$ also may be prepared by methods known in the art, for example, when $R^1$ is as hereinbefore defined, by the reaction of $NO_2R^{18}$, wherein $R^{18}$ is

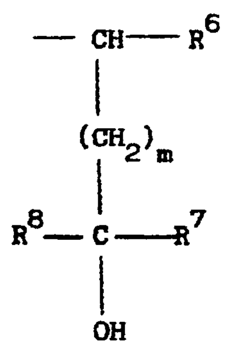

wherein $R^6$ to $R^8$ and m are as hereinbefore defined, with an appropriate aldehyde, conveniently acetaldehyde or formaldehyde (as in B.M. Vanderbilt and H.B. Hass, Ind. Eng. Chem. 32, 34 (1940)) followed by reduction (as outlined in J. March, vide supra, pages 1125-1126), conveniently by hydrogen and a metal catalyst (for example, a platinum containing catalyst) in an appropriate solvent, conveniently glacial acetic acid.

2. The reduction of a compound Ar. CO. $NHR^1$

wherein Ar and $R^1$ are as hereinbefore defined and the hydroxy groups are optionally protected, followed by deprotection of the hydroxy groups where appropriate. The reduction may be carried out by standard reducing agents known for carrying out this type of reduction that will not reduce the aromatic ring system (as outlined in J. March, vide supra, page 1122), for example, a hydride reagent such as lithium aluminium hydride in an inert solvent, such as an ether, i.e. tetrahydrofuran, at a non-extreme temperature, for example, at between 0° and 100°C and conveniently at the reflux temperature of the ether.

The compound Ar. CO. $NHR^1$ may be formed by the reaction of the appropriate acid (ArCOOH) or a suitable reactive acid derivative thereof as outlined in J. March, vide supra, pages 382-390) for example, an acid halide in an inert solvent, with an amine $NH_2R^1$ in which the hydroxy groups are optionally protected, for example, when the compound $NH_2R^1$ is a diol, by an isopropylidene group. The compound Ar. CO. $NHR^1$ so formed is suitably reduced in situ and deprotected if necessary to give a compound of formula (I). The compounds of the formula ArCOOH can be prepared by methods well known to those skilled in the art.

3. The reaction of a compound $ArCH_2L$ (wherein Ar is as hereinbefore defined and L is a leaving group), with a compound $NH_2R^1$ as hereinbefore defined. Suitable leaving groups are those defined by J. March, vide supra pages 325–331, and include halogens such as chlorine or bromine and sulfonic acid derivatives such as p-toluenesulfonate. The reaction is suitably carried out in an appropriate solvent, such as a dipolar aprotic solvent or alcohol at a non-extreme temperature, for example 50–100°. The compounds of the formula $ArCH_2L$ can be prepared by methods well known to those skilled in the art, for example in the case of a compound where L is bromo by the reaction of a compound $ArCH_3$ with N-bromosuccinamide or a similar reagent. Compounds of the formula $ArCH_3$ are prepared in a manner analogous to that used for the preparation of the parent hydrocarbon with the starting materials appropriately substituted by a methyl group.

The claimed compounds can be prepared by any method known for the preparation of analogous compounds, in particular those methods defined in (1) to (3) hereinabove.

The compounds of this invention have biocidal activity, e.g. are toxic to certain living cells which are detrimental to mammals, for example pathogenic organisms and tumours.

This toxicity to pathogenic organisms has been demonstrated for example, by activity against one or more of the following: viruses (e.g. Herpes simplex l/vero), fungi (e.g. Candida albicans), protozoa (e.g. Eimeria tenella and Irichomonas vaginalis), bacteria (e.g. Mycoplasma smeg matis and Streptococcus pyogenes), and helminths (e.g. Nippostrongylus brasiliensis and Brugia pahangi). The antitumour activity

of compounds of formula I has been demonstrated in a number of recognized screens and primarily by activity against ascitic P388/0 leukaemia.

Preferred compounds of the formula (I) are those which have antitumour activity. The activity against ascitic tumours, including P388/0, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/0, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour in man (A. Goldin et al. in Methods in Cancer Research ed. V.T. DeVita Jr. and H. Busch, 16, 165, Academic Press, N.Y. 1979).

There are sublines of P388/0 which have been made resistant to the following clinically useful agents: cytosine arabinoside, doxorubicin, cyclophosphamide, L-phenylalanine mustard, methotrexate, 5-fluorouracil, actinomycin D, cis-platin and bis-chloroethylnitrosourea. Compounds of this invention show potent activity against these drug-resistant tumours using the procedure for P388/0 above.

Compounds of the formula (I) have also been found to be active against human tumour cells in primary cultures of lung, ovary, breast, renal, melanoma, unknown primary, gastric, pancreatic, mesothelioma, myeloma and/or colon cancer. (As used herein "cancer" is to be taken as synonymous with "malignant tumour" or more generally "tumour" unless otherwise noted). This is a procedure in which the prevention of tumour cell colony formation, i.e. tumour cell replication, by a drug has been shown to correlate with clinical antitumour activity in man (D.D. Von Hoff et al., Cancer Chemotherapy and Pharmacology 6, 265 (1980); S. Salmon and D.D. Von Hoff, Seminars in Oncology, 8, 377 (1981)).

Compounds of formula I which have been found to have antitumour activity intercalate in vitro with DNA (this property is determined by viscometric methods using the procedure of W.D. Wilson et al., Nucleic Acids Research 4, 2697 (1954) and have a log P as calculated by the method of C. Hansch and A. Leo in Substituent Constants for Correlation Analysis in Chemistry and Biology, John Wiley and Sons, New York, 1979, lying in the range between −2.0 and +2.5.

As has been described above, the compounds of the present invention are useful for the treatment of tumours in animals, including mammals, especially humans, by the administration of a clinically useful amount of compound of formula (I) in a pharmaceutically useful form, once or several times a day or other appropriate schedule, orally, rectally, parenterally, or applied topically. The invention further provides a compound of formula (I) for use in therapy, for example as an antitumour agent.

The amount of compound of formula (I) required to be effective as a biological agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, e.g., two to six times per day or by intravenous infusion for selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical formulation. Formulations of the present invention, for medical use, comprise an active compound together with one or more pharmaceutically acceptable carriers thereof and optionally other therapeutical ingredients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester or pharmaceutically acceptable salt thereof, and a pharmaceutically acceptable carrier therefor.

Whilst the antitumour activity of the compounds of formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated aqueous solution of a sugar, for example sucrose, to which may also be added any accessory ingredients. Such accessory ingredient(s) may include flavourings, an agent to retard crystallization of the sugar or an agent to increase the solubility of any other ingredient, such as a polyhydric alcohol for example glycerol or sorbitol.

Formulations for rectal administration may be presented as a suppository with a conventional carrier such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

General Comments

All solvents were reagent grade and used without further purification with the following exceptions. Tetrahydrofuran (THF) was dried by distillation from Na/K alloy under nitrogen ($N_2$) and used immediately. Toluene ($PhCH_3$) was distilled from $CaH_2$ under $N_2$ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plugs of $SiO_2$ used for purifications were «flash chromatography» silica gel (Merck Co., Inc., Merck Chemical Division, Rahway, NJ, 07065 silica gel 60, 230–400 mesh). An appropriate volume sintered glass funnel was filled approximately 3/4 full with the silica gel and packed evenly by tapping the outside of the funnel. A piece of filter paper was then placed on top of the silica gel and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents and elemental analyses (all elements analyzing within a difference of $\pm$0.4% of the expected value). Any changes to the procedure such as solvent, reaction temperature, reaction time, or workup are noted.

NMR ($^1H$, $^{13}C$), IR, MS data of all new products were consistent with the expected and proposed structures. The positions assigned to structural isomers were unequivocally determined by a number of NMR techniques. All final products were dried in a vacuum oven at 20mm Hg (26.7 mbar or 2666.4 Pa) pressure at the temperature indicated overnight (12–16 hours). All temperatures are in degrees Celsius.

Example 1

2-Methyl-2-[[(3-methyl-3H-naphth[2,3-e]indol-11-yl) methyl]amino]-1,3-propanediol

1A 3-Methyl-3H-naphth[2,3-e]indole-11- carbaldehyde

To a round bottom flask equipped with overhead stirrer, condenser, $N_2$ inlet line was added $CH_2Cl_2$ (500 ml), and $SnC1_4$ (Aldrich, 14.1 g, 6.3 ml, 0.054 mol). To the flask was then added , -dichloromethylmethylether (Aldrich, 6.2 g, 4.9ml, 0.054mol) dropwise over 10 min. Ethyl 3-methyl-3H-naphth[2,3-e]indole-2-carboxylate (H.G. Pars Pharmaceutical Laboratories, 763 Concord Avenue, Cambridge, MA, 02138, 13.01 g, 0.043 mol) was added in one portion as a solid to the flask. After 30 min a dark red solid had deposited in the flask. After 3 hr the mixture was refluxed for 15 min. cooled and treated with saturated NaOAc solution (200 ml) and stirred for an additional 30 min. After addition of 31 of $CH_2Cl_2$ the material was completely dissolved. The organic layer was dried and passed through a plug of silica gel using $CH_2Cl_2$ followed by EtOAc as the eluting solvents to remove polar materials. The appropriate fractions were combined and the solvent removed to give 12.4g of the crude aldehyde ethyl ester mixture. This material was then suspended in a mixture of THF (200 ml) and $H_2O$ (200 ml) and 1N NaOH solution (50 ml) and refluxed for 30 min. The crude aldehyde acid salt mixture was then cooled and poured into 1l of 1N HCl solution and the resulting crude acid filtered, washed with $H_2O$ (200 ml) and dried overnight in a vacuum oven at 100° to give 11.7 g of crude aldehyde acid mixture. This was then decarboxylated without further purification. To a flask containing the crude product was added Cu (Fisher, 6.5g, 0.102 mol) and quinoline (Aldrich, 100 ml). The mixture was heated at reflux for 30 min. cooled and diluted with EtOAc (500 ml). The mixture was extracted with 0.1 N HCl solution (3x500 ml) dried ($Na_2SO_4$) and filtered to give a clear dark yellow solution. The solvent was removed to give the solid which then chromatographed on silica gel using $CH_2Cl_2$ as the solvent. The appropriate fractions were combined and the solvent removed to give the major aldehyde component. Crystallization of this material ($CH_2Cl_2$/petroleum ether) gave a total of 4.16g (36.4% from the ester) of 3-methyl-3H-naphth[2,3-e]indole-11-carbaldehyde, mp 175-176°, (C, H, N).

1B 2-Methyl-2-[[(3-methyl-3H-naphth[2,3-e]indol-11-yl)methyl]amino]-1,3-propanediol

To a round bottom flask equipped with magnetic stirring bar, condenser, Dean-Stark trap and $N_2$ bubbler was added 3-methyl-3H-naphth[2,3-e]-indole -11-carbaldehyde (1A, 4.15 g 0.016 mol), 2-amino-2-methyl-1,3-propanediol (Aldrich, 3.37g 0.032 mol), p-toluenesulfonic acid (10mg) and toluene (250ml). The reaction was heated at reflux with azeotropic removal of $H_2O$ for 5hr and then most of the toluene was removed by distillation until approximately 100ml remained in the flask. The mixture was cooled and 200ml of absolute EtOH added followed by $NaBH_4$ (MC&B, 1.21g, 0.032 mol).

The reaction was then stirred at room temperature overnight. The solvent was then removed by rotary evaporation and the resulting orange solid shaken with warm $H_2O$ (250ml). The solid was then filtered and washed with additional $H_2O$ (250ml) and then dried in a vacuum oven at 100°. The material was then dissolved in a mixture of EtOH and dry HCl gas (400 ml), filtered through a fritted glass funnel and diluted to 2l with $Et_2O$. The yellow solid which formed was then filtered, washed with $Et_2O$ (300ml) and crystallized twice from $CH_3OH/Et_2O$ (250/750 ml), filtered, washed with more $Et_2O$ and dried overnight in a vacuum oven at 80°. There was obtained 4.29g (69.7%) of 2-methyl-2-[[(3-methyl-3H-naphth[2,3-e]indol-11-yl)-methyl] amino]-1,3-propanediol hydrochloride 1/2 $H_2O$, mp 200° (turns black but does not melt below 300°), (C, H, N, Cl).

Example 2

2-Methyl-2-[[(1-methyl-1H-naphth[2,3-g]indol-5-yl)methyl]amino]-1,3-propanediol

2A 1-Methyl-1H-naphth[2,3-g]indole-3-carbaldehyde

2B 1-Methyl-1H-naphth[2,3-g]indole-5-carbaldehyde

2C 1-Methyl-1H-naphth[2,3-g]indole-6-carbaldehyde.0.1 $H_2O$

Using the procedure described in Example 1A, ethyl 1-methyl-1H-naphth [2,3-g]indole-2-carboxylate (H.G.Pars Pharmaceutical Laboratories) gave a mixture of aldehydes which was purified by $SiO_2$ chromatography using $CH_2Cl_2$ for the first column then $PhCH_3/CH_2Cl_2$ mixtures for subsequent columns. Three aldehydes were obtained pure in this way:

1-methyl-1H-naphth[2,3-g]indole-3-carbaldehyde (2A), mp 182-183° ($PhCH_3$), (C,H,N), $R_f = 0.11$ ($SiO_2/CH_2$), 22.2% yield;

1-methyl-1H-naphth[2,3-g]indole-5-carbaldehyde(2B), mp 128-131°, ($PhCH_3$), (C,H,N),

$R_f$ = 0.32($SiO_2/CH_2Cl_2$), 32.3% yield;
1-methyl-1H-naphth[2,3-g]indole-6-carbaldehyde.0.1 $H_2O$ (2C), mp 156.5-159°, isolated directly from column, (C,H,N), $R_f$ = 0.5 ($SiO_2/CH_2Cl_2$), 4.4% yield.

2D 2-Methyl-2[[(1-methyl-1H-naphth[2,3-g]indol-5-yl)-methyl]amino]-1,3-propanediol hydrochloride .0.25 $H_2O$

Using the procedure outlined in Example 1, 1-methyl-1H-naphth[2,3-g] indole-5-carbaldehyde (2B) and 2-amino-2-methyl-1,3-propanediol (Aldrich) gave a 61.1% yield of 2-methyl-2-[[(1-methyl-1H-naphth[2,3-g] indol-5-yl)methyl]amino]-1,3-propanediol hydrochloride .0.25 $H_2O$, mp approx 300° (dec, darkens at 264°), EtOH/$Et_2O$), (C,H,N,Cl).

Antitumour Screening Results

Methods for evaluating the antitumour activity of these compounds are essentially those used in the Tumor Panel by the Development Therapeutics Program, Division of Cancer Treatment, National Cancer Institute, A. Goldin, et al., Methods in Cancer Research, Vol. XVI, p. 165, Academic Press (1979). Some modifications, in dose level and schedule have been made to increase the testing efficiency.

Lymphocytic Leukemia P388/0 Test

CD2-$F_1$ mice, of the same sex weighing within 20±3g, are used for this test. Control and test animals are injected intraperitoneally with a suspension of $10^6$ viable P388/0 tumor cells on day 0. In each test several dose levels which bracket the $LD_{20}$ for the compound are evaluated; each dose level group contains 6 animals. The test compounds are prepared either in physiologic saline containing 0.05% Tween 80 or distilled water containing 5% dextrose and are administered intraperitoneally on days, 1, 5, and 9 relative to tumor implant. Doses are on a mg/kg basis according to individual animals' body weights. The day of death for each animal is recorded, the median identified for each group and the ratios of median survival time for treated (T)/control (C) groups are calculated. The criterion for activity is T/C x 100 ≧120%. Results of P388/0 testing are summarized below.

2-Methyl-2-[[(3-methyl-3H-naphth[2,3-e]indol-11-yl)methyl]amino]1,3-propanediol hydrochloride gave a T/C value of 250% and 3 out of 6 30 day survivors at a dose of 50 mg/kg

2-Methyl-2-[[(1-methyl-1H-naphth[2,3-g]indol-5-yl)methyl]amino]-1,3-propanediol hydrochloride gave a T/C value of 145%, $LD_{20}$ = 50 at a dose of 55 mg/kg

Formulation Examples

A. TABLET

| | |
|---|---|
| Compound of Formula I (as methanesulphonate)) | 500.0 mg |
| Pregelatinised Corn Starch | 60.0 mg |
| Sodium Starch Glycolate | 36.0 mg |
| Magnesium Stearate | 4.0 mg |

The compound of formula (I) is finely ground and intimately mixed with the powdered excipients, pregelatinised corn starch and sodium starch glycolate. The powders are wetted with purified water to form granules. The granules are dried and mixed with the magnesium stearate. The formulation is then compressed into tablets weighing approximately 600 mg each.

B. TABLET

| | |
|---|---|
| Compound of formula (I) | 500.0 mg |
| Corn Starch | 70.0 mg |
| Lactose | 83.8 mg |
| Magnesium Stearate | 4.2 mg |
| Polyvinylpyrrolidone | 14.0 mg |
| Stearic Acid | 28.0 mg |

The Compound of formula (I) is finely ground and intimately mixed with the powdered excipients, corn starch and lactose. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 700 mg each.

C. CAPSULES

| | |
|---|---|
| Compound of formula (I) | 500.0 mg |
| Corn Starch | 50.0 mg |
| Magnesium Stearate | 3.0 mg |

The finely divided compound of formula (I) is mixed with powdered corn starch and wetted with denatured alcohol to densify the powder. The dried powder is mixed with stearic acid and filled into hard-shell gelatin capsules.

D. SYRUP

| | |
|---|---|
| Compound of formula (I) | 250.0 mg |
| Ethanol | 250.0 mg |
| Glycerin | 500.0 mg |
| Sucrose | 3,500.0 mg |
| Flavouring Agent | q.s. |
| Colouring Agent | q.s. |
| Presserving Agent | 0.1% |
| Purified Water | q.s. to 5.0 ml |

The Compound of formula (I) is dissolved in the ethanol, glycerin, and a portion of the purified water. The sucrose and preserving agent are dissolved in another portion of hot purified water, and then the coloring agent is added and dissolved. The two solution are mixed and cooled before the flavouring agent is added. Purified water is added to final volume. The resulting syrup is thoroughly mixed.

E. IV INJECTION

| | |
|---|---|
| Compound of formula (I) (as methanesulphonate) | 5.0 mg |
| Glycerin | q.s. for isotonicit |
| Preservative | 0.1% |
| Hydrochloric Acid of | as needed for |
| Sodium Hydroxide | pH adjustment |
| Water for Injection | q.s. to 1 ml. |

The compound of formula (I) and preservative is added to the glycerin and a portion of the water for injection. The pH is adjusted with hydrochloric acid or sodium hydroxide. Water for injection is added to final volume and solution is complete after thorough mixing. The solution is sterilised by filtration through a 0.22 micrometer membrane filter and aseptically filled into sterile 10 ml ampoules or vials.

**Claims for the Contracting States: ES, AT, GR**

1. A process for the preparation of a compound of the formula (I):

$ArCH_2NHR^1$ (I)

or a monomethyl or monoethyl ether thereof, the compound of formula (I) including the said ethers containing no more than 29 carbon atoms in total; an ester thereof; a salt thereof;
wherein Ar is a ring system of formula

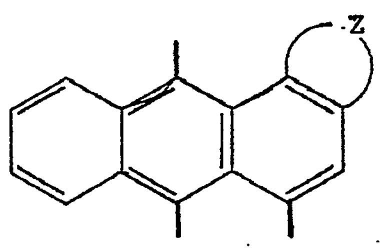

wherein the $CH_2NHR_1$ side chain is attached to one of the inticated positions,
wherein

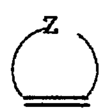

is a five-membered ring containing two double bonds, and Z is oxygen, sulphur or nitrogen substituted by hydrogen, methyl or ethyl or a resonance form thereof, Ar being optionally substituted by one or two substituents, said substituents containing not more than four carbon atoms in total when taken together and being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0,1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five-or six-membered heterocyclic ring optionally containing one or two additional heteroatoms;
$R^1$ contains not more than eight carbon atoms and is a group

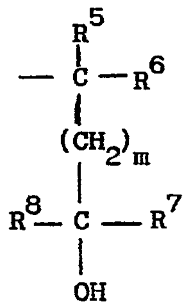

or

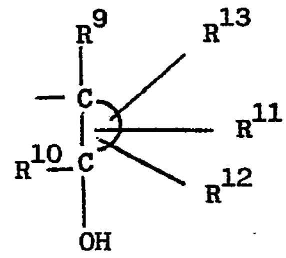

wherein m is 0 or 1;
$R^5$ and $R^6$ are the same or different and each is hydrogen or $C_{1-5}$ alkyl optionally substituted by hydroxy;
$R^7$ and $R^8$ are the same or different and each is hydrogen or $C_{1-3}$ alkyl;
-C-C is a five-or-six-membered saturated carbocyclic ring;
$R^9$ is hydrogen, methyl or hydroxymethyl;
$R^{10}$, $R^{11}$ and $R^{12}$ are the same or different and each is hydrogen or methyl;
$R^{13}$ is hydrogen, methyl, hydroxy, or hydroxymethyl,
which process comprises:

(a) the reduction of a compound $ArCH{=}NR^1$; or an appropriately protected derivative thereof, followed by deprotection where appropriate;

(b) the reduction of a compound $Ar.CO.NHR^1$, wherein the hydroxy groups are optionally protected, followed by deprotection of the hydroxy groups where appropriate;

(c) the reaction of a compound $ArCH_2L$, wherein L is a leaving group, with a compound $NH_2R^1$.

2. A process according to claim 1 for the preparation of a compound wherein the $CH_2NHR^1$ side chain is attached at one of the positions indicated below:

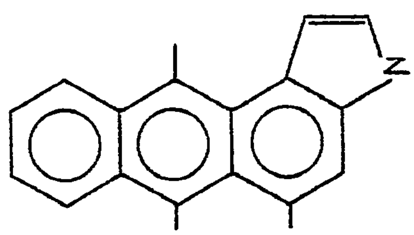

wherein Z=S, O, NMe, or NEt.

3. A process according to claim 1 for the preparation of a compound wherein Ar is 2-Methyl-2-[[(3-methyl-3H-naphth[2,3,-e]indol-11-yl)-methyl]amino]-1,3-propanediol or acid addition salts thereof.

4. A process according to claim 1 for the preparation of a compound wherein $R^1$ is

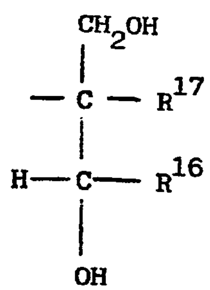

wherein $R^{16}$ is hydrogen or methyl and $R^{17}$ is hydrogen, methyl or ethyl.

5. A pharmaceutical formulation comprising a compound of the formula (I), an ether, ester or pharmaceutically acceptable acid addition salt thereof, as defined in claim 1, and a pharmaceutically acceptable carrier therefor.

**Claims for the contracting states: BE, CH, DE, FR, GB, IT, LI, LU, NL, SE**

1. A compound of the formula (I):

$ArCH_2NHR^1$ (I)

or a monomethyl or monoethyl ether thereof, the compound of formula (I) including the said ethers containing no more than 29 carbon atoms in total; an ester thereof; a salt thereof;

wherein Ar is a ring system of formula

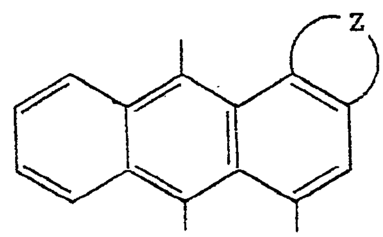

wherein the $CH_2NHR_1$ side chain is attached to one of the indicated positions, wherein

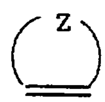

is a five-membered ring containing two double bonds, and Z is oxygen, sulphur or nitrogen substituted by hydrogen, methyl or ethyl or a resonance form thereof, Ar being optionally substituted by one or two substituents, said substituents containing not more than four carbon atoms in total when taken together and being the same or different and are selected from halogen: cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0,1 or 2 and $R^2$ is $C_{1-2}$ alkyl optionally substituted by hydroxy or $C_{1-2}$ alkoxy: or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five-or six-membered heterocyclic ring optionallv containing one or two additional heteroatoms;

$R^1$ contains not more than eight carbon atoms and is a group

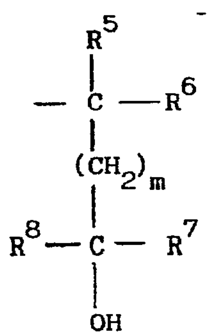

or

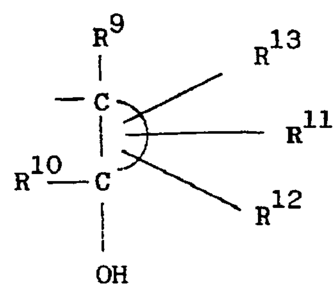

wherein m is 0 or 1;

$R^5$ and $R^6$ are the same or different and each is hydrogen or $C_{1-3}$ alkyl optionally substituted by hydroxy;

$R^7$ and $R^8$ are the same or different and each is hydrogen or $C_{1-3}$ alkyl; –C–C is a five-or-six-membered saturated carbocyclic ring;

$R^9$ is hydrogen, methyl or hydroxymethyl;

$R^{10}$, $R^{11}$ and $R^{12}$ are the same or different and each is hydrogen or methyl;

$R^{13}$ is hydrogen, methyl, hydroxy, or hydroxymethyl.

2. A compound according to claim 1 wherein the $CH_2NHR^1$ side chain is attached at one of the positions indicated below:

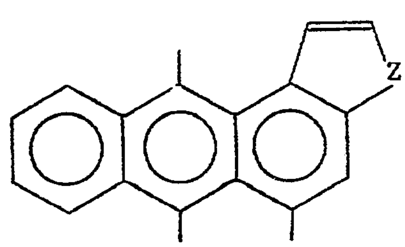

wherein Z= S, O NMe or NEt

3. A compound according to claim 1 which is 2-Methyl-2[[3-methyl-3H-naphth[2,3-e]indol-11-yl)methyl]amino]-1,3-propanediol or acid addition salts thereof.

4. A compound according to claim 1 wherein $R^1$ is

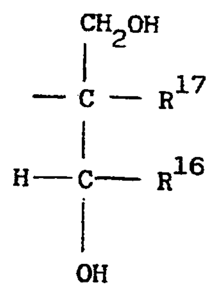

wherein $R^{16}$ is hydrogen or methyl and $R^{17}$ is hydrogen, methyl or ethyl.

5. A pharmaceutical formulation comprising a compound as defined in any of claims 1 to 4 and a pharmaceutically acceptable carrier therefor.

6. A process for the preparation of a compound of the formula (I), as defined in claim 1, which process comprises.

(a) the reduction of a compound $ArCH=NR^1$ or an appropriately protected derivative thereof, followed by deprotection where appropriate;

(b) the reduction of a compound $Ar.CO.NHR^1$, wherein the hydroxy groups are optionally protected, followed by deprotection of the hydroxy groups where appropriate;

(c) the reaction of a compound $ArCH_2L$, wherein L is a leaving group, with a compound $NH_2R^1$.

7. A compound of the formula (I) or an ether, ester or pharmaceutically acceptable acid addition salt thereof, as defined in claim 1, for use in the treatment of tumours.

8. A compound of the formula (I) or an ether, ester or pharmaceutically acceptable acid addition salt thereof, as defined in claim 1, for use in the preparation of a medicament for the treatment of tumours.

**Patentansprüche für die Vertragsstaaten: BE, CH, DE, FR, GB, IT, LI, LU, NL, SE**

1. Verbindung der Formel (I):

$ArCH_2NHR^1$ (I)

oder ein Monomethyl- oder Monoäthyläther hievon, wobei die Verbindung der Formel (I) diese Äther mit nicht mehr als insgesamt 29 Kohlenstoffatomen einschließt; ein Ester hievon und ein Salz hievon; worin Ar ein Ringsystem der Formel

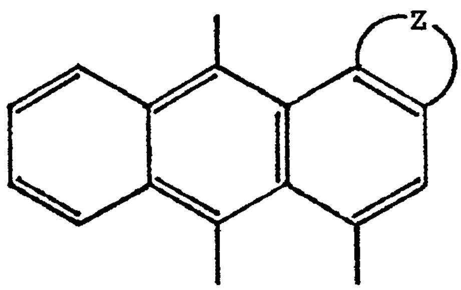

darstellt und sich die Seitenkette $CH_2NHR^1$ in einer der angegebenen Stellungen befindet, wobei

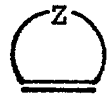

einen 5-gliedrigen Ring mit zwei Doppelbindungen bedeutet und Z Sauerstoff, Schwefel oder Stickstoff substituiert durch Wasserstoff, Methyl oder Äthyl oder eine Resonanzform hievon ist, wobei Ar gegebenenfalls durch einen oder zwei Substituenten substituiert ist und diese Substituenten miteinander nicht mehr als vier Kohlenstoffatome enthalten und gleich oder verschieden sind und ausgewählt sind aus Halogen; Cyano; $C_1$–$C_4$-Alkyl oder C1–C4-Alkoxy, jeweils gegebenenfalls substituiert durch Hydroxy oder $C_1$–$C_2$-Alkoxy; halogensubstituiertem C1–C2-Alkyl oder C1–C2-Alkoxy; einer Gruppe $(S(O)_nR^2$, worin n eine ganze Zahl von Null, 1 oder 2 ist und $R^2$ $C_1$–$C_2$-Alkyl gegebenenfalls substituiert

durch Hydroxy oder $C_1$–$C_2$-Alkoxy bedeutet; oder Ar gegebenenfalls durch eine Gruppe $NR^3R^4$ mit nicht mehr als 5 Kohlenstoffatomen substituiert ist, wobei $R^3$ und $R^4$ gleich oder verschieden sind und jeweils eine $C_1$–$C_3$-Alkylgruppe bedeuten oder $NR^3R^4$ einen fünf- oder sechsgliedrigen heterocyclischen Ring bildet, der gegebenenfalls ein oder zwei weitere Heteroatome enthält;

$R^1$ nicht mehr als acht Kohlenstoffatome enthält und eine Gruppe

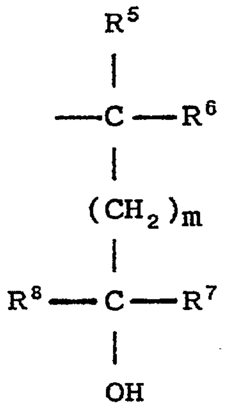

oder

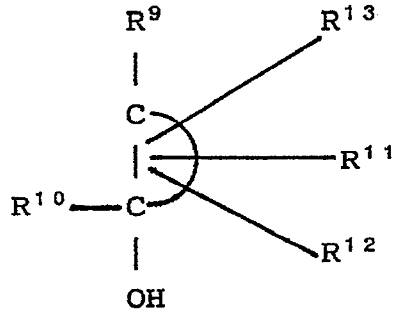

darstellt, wobei m Null oder 1 ist;

$R^5$ und $R^6$ gleich oder verschieden sind und jeweils Wasserstoff oder gegebenenfalls hydroxysubstituiertes $C_1$–$C_5$-Alkyl bedeuten; $R^7$ und $R^8$ gleich oder verschieden sind und jeweils Wasserstoff oder $C_1$–$C_3$-Alkyl darstellen;

–C–C ein fünf- oder sechsgliedriger gesättigter carbocyclischer Ring ist;

$R^9$ Wasserstoff, Methyl oder Hydroxymethyl bedeutet;

$R^{10}$, $R^{11}$ und $R^{12}$ gleich oder verschieden sind und jeweils Wasserstoff oder Methyl darstellen; und $R^{13}$ Wasserstoff, Methyl, Hydroxy oder Hydroxymethyl ist.

2. Verbindung nach Anspruch 1, worin sich die Seitenkette $CH^2NHR_1$ an einer der im nachstehenden angegebenen Stellungen befindet:

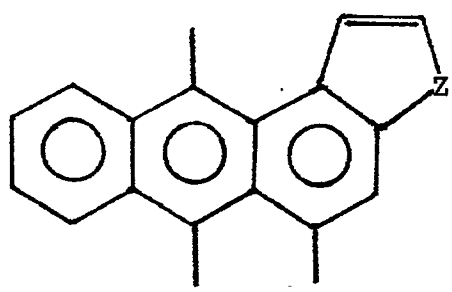

worin Z = S, O, NMe oder NÄt bedeutet.

3. Verbindung nach Anspruch 1, die 2-Methyl-2[[3-methyl-3H-naphth[2,3-e]indol-11-yl)methyl]amino]-1,3-propandiol oder ein Säureadditionssalz hievon ist.

4. Verbindung nach Anspruch 1, worin $R^1$ die Bedeutung

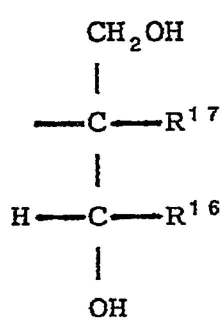

hat, wobei $R^{16}$ Wasserstoff oder Methyl bedeutet und $R^{17}$ Wasserstoff, Methyl oder Äthyl darstellt.

5. Pharmazeutische Formulierung umfassend eine Verbindung nach einem der Ansprüche 1 bis 4 und einen pharmazeutisch annehmbaren Träger hiefür.

6. Verfahren zur Herstellung einer Verbindung der Formel (I), wie in Anspruch 1 definiert, welches Verfahren umfaßt:
(a) die Reduktion einer Verbindung $ArCH{=}NR^1$ oder eines geeignet geschützten Derivates hievon, wenn geeignet, gefolgt von der Entfernung der Schutzgruppe(n);
(b) die Reduktion einer Verbindung $Ar.CO.NHR^1$, worin die Hydroxygruppen gegebenenfalls geschützt sind, wenn geeignet, gefolgt von der Entfernung der Schutzgruppe(n) der Hydroxygruppen;
(c) die Umsetzung einer Verbindung $ArCH_2L$, worin L eine abspaltbare Gruppe ist, mit einer Verbindung $NH_2R^1$.

7. Verbindung der Formel (I) oder ein Äther, Ester oder pharmazeutisch annehmbares Säureadditionssalz hievon, wie in Anspruch 1 definiert, zur Verwendung bei der Behandlung von Tumoren.

8. Verbindung der Formel (I) oder ein Äther, Ester oder pharmazeutisch annehmbares Säureadditionssalz hievon, wie in Anspruch 1 definiert, zur Verwendung bei der Herstellung eines Medikaments zur Behandlung von Tumoren.

**Patentansprüche für die Vertragsstaaten: AT, ES, GR**

1. Verfahren zur Herstellung einer Verbindung der Formel (I):

$ArCH_2NHR^1$ (I)

oder eines Monomethyl- oder Monoäthyläthers hievon, wobei die Verbindung der Formel (I) diese Äther mit nicht mehr als insgesamt 29 Kohlenstoffatomen einschließt; eines Esters hievon und eines Salzes hievon; worin Ar ein Ringsystem der Formel

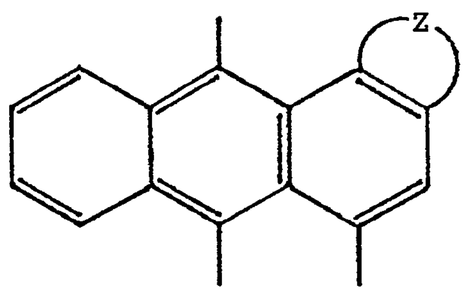

darstellt und sich die Seitenkette $CH_2NHR^1$ in einer der angegebenen Stellungen befindet, wobei

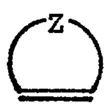

einen 5-gliedrigen Ring mit zwei Doppelbindungen bedeutet und Z Sauerstoff, Schwefel oder Stickstoff substituiert durch Wasserstoff, Methyl oder Äthyl oder eine Resonanzform hievon ist, wobei Ar gegebenenfalls durch einen oder zwei Substituenten substituiert ist und diese Substituenten miteinander nicht mehr als vier Kohlenstoffatome enthalten und gleich oder verschieden sind und ausgewählt sind aus Halogen; Cyano; $C_1$–$C_4$-Alkyl oder $C_1$–$C_4$-Alkoxy, jeweils gegebenenfalls substituiert durch Hydroxy oder $C_1$–$C_2$-Alkoxy; halogensubstituiertem $C_1$–$C_2$-Alkyl oder $C_1$–$C_2$-Alkoxy; einer Gruppe $(S(O)_nR^2$, worin n eine ganze Zahl von Null, 1 oder 2 ist und $R^2$ $C_1$–$C_2$-Alkyl gegebenenfalls substituiert durch Hydroxy oder $C_1$–$C_2$-Alkoxy bedeutet; oder Ar gegebenenfalls durch eine Gruppe $NR^3R^4$ mit nicht mehr als 5 Kohlenstoffatomen substituiert ist, wobei $R^3$ und $R^4$ gleich oder verschieden sind und jeweils eine $C_1$–$C_3$-Alkylgruppe bedeuten oder $NR^3R^4$ einen fünf- oder sechsgliedrigen heterocyclischen Ring bildet, der gegebenenfalls ein oder zwei weitere Heteroatome enthält;
$R^1$ nicht mehr als acht Kohlenstoffatome enthält und eine Gruppe

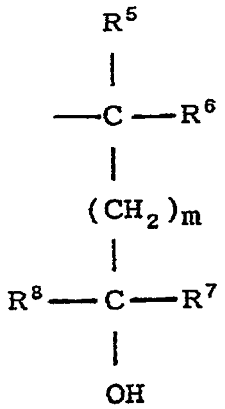

oder

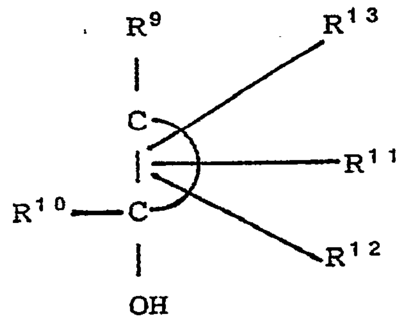

darstellt, wobei m Null oder 1 ist;
$R^5$ und $R^6$ gleich oder verschieden sind und jeweils Wasserstoff oder gegebenenfalls hydroxysubstituiertes $C_1$–$C_5$-Alkyl bedeuten; $R^7$ und $R^8$ gleich oder verschieden sind und jeweils Wasserstoff oder $C_1$–$C_3$-Alkyl darstellen;
–C–C ein fünf- oder sechsgliedriger gesättigter carbocyclischer Ring ist;
$R^9$ Wasserstoff, Methyl oder Hydroxymethyl bedeutet;
$R^{10}$, $R^{11}$ und $R^{12}$ gleich oder verschieden sind und jeweils Wasserstoff oder Methyl darstellen; und $R^{13}$ Wasserstoff, Methyl, Hydroxy oder Hydroxymethyl ist, welches Verfahren umfaßt:

(a) die Reduktion einer Verbindung $ArCH=NR^1$ oder eines geeignet geschützten Derivates hievon, wenn geeignet, gefolgt von der Entfernung der Schutzgruppe(n);

(b) die Reduktion einer Verbindung $Ar.CO.NHR^1$, worin die Hydroxygruppen gegebenenfalls geschützt sind, wenn geeignet, gefolgt von der Entfernung der Schutzgruppe(n) der Hydroxygruppen;

(c) die Umsetzung einer Verbindung $ArCH_2L$, worin L eine abspaltbare Gruppe ist, mit einer Verbindung $NH_2R^1$.

2. Verfahren nach Anspruch 1 zur Herstellung einer Verbindung, worin sich die Seitenkette $CH_2NHR^1$ an einer der im nachstehenden angegebenen Stellungen befindet:

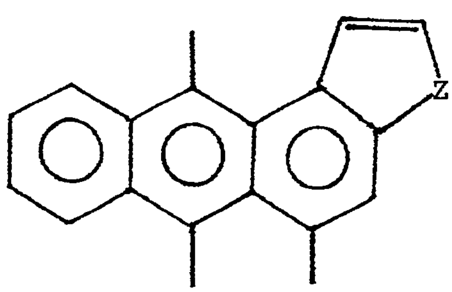

worin Z = S, O, NMe oder NÄt bedeutet.

3. Verfahren nach Anspruch 1 zur Herstellung der Verbindung 2-Methyl-2[[3-methyl-3H-naphth[2,3-e]indol-11-yl)methyl]amino]-1,3-propandiol oder eines Säureadditionssalzes hievon.

4. Verfahren nach Anspruch 1 zur Herstellung einer Verbindung, worin $R^1$ die Bedeutung

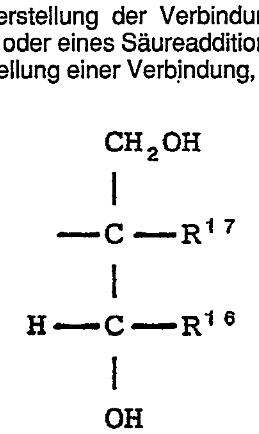

hat, wobei $R^{16}$ Wasserstoff oder Methyl bedeutet und $R^{17}$ Wasserstoff, Methyl oder Äthyl darstellt.

5. Pharmazeutische Formulierung umfassend eine Verbindung der Formel (I), einen Äther, Ester oder ein pharmazeutisch annehmbares Säureadditionssalz hievon, wie in Anspruch 1 definiert, und einen pharmazeutisch annehmbaren Träger hiefür.

**Revendications pour les Etats contractants: BE, CH, DE, FR, GB, IT, LI, LU, NL, SE**

1. Composé de formule (I):

$ArCH_2NHR^1$ (I)

ou un éther monométhylique ou monoéthylique de celui-ci; le composé de formule (I) y compris ces éthers ne comptant pas plus de 29 atomes de carbone au total; un ester de celui-ci; un sel de celui-ci; où Ar est un système cyclique de formule :

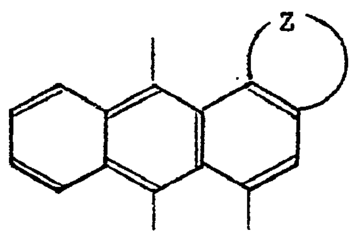

où la chaîne latérale $CH_2NHR^1$ est attachée à l'une des positions indiquées, où

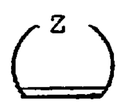

est un cycle de cinq chaînons contenant deux doubles liaisons et Z est oxygène, soufre ou azote substitué parhydrogène, méthyle ou éthyle, ou une forme de résonance de celui-ci,
Ar portant éventuellement un ou deux substituants, ces substituants ne comptant pas plus de quatre atomes de carbone au total lorsqu'ils sont pris ensemble et étant identiques et différents et choisis parmi halogène; cyano; $C_{1-4}$-alcoyle ou $C_{1-4}$-alcoxy, chacun éventuellement substitués par hydroxyle ou $C_{1-2}$-alcoxy; $C_{1-2}$-alcoyle ou $C_{1-2}$-alcoxy halogéno-substitué; un radical $S(O)_nR^2$, où n est un entier 0,1 ou 2 et $R^2$ est $C_{1-2}$-alcoyle éventuellement substitué par hydroxyle ou $C_{1-2}$-alcoxy; ou bien Ar est éventuellement substitué par un radical $NR^3R^4$ ne comptant pas plus de 5 atomes de carbone, où $R^3$ et $R^4$ sont identiques ou différents et sont chacun un radical $C_{1-3}$-alcoyle ou $NR^3R^4$ forme un hétérocycle de cinq ou six chaînons contenant éventuellement un ou deux hétéroatomes supplémentaires;
$R^1$ ne compte pas plus de huit atomes de carbone et est un radical:

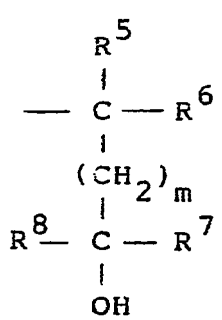

ou

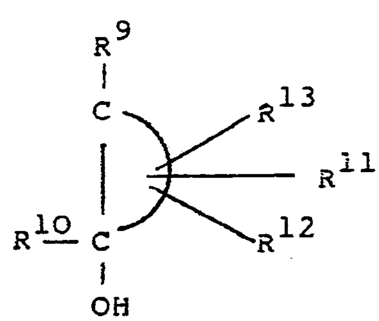

où est 0 ou 1; $R^5$ et $R^6$ sont identiques ou différents et sont chacun hydrogène ou $C_{1-3}$-alcoyle éventuellement substitué par hydroxyle; $R^7$ et $R^8$ sont identiques ou différents et sont chacun hydrogène ou $C_{1-3}$-alcoyle; –C–C est un carbocycle saturé de cinq ou six chaînons; $R^9$ est hydrogène, méthyle ou hydroxyméthyle; $R^{10}$, $R^{11}$ et $R^{12}$ sont identiques ou différents et sont chacun hydrogène ou méthyle; $R^{13}$ est hydrogène, méthyle, hydroxyle ou hydroxyméthyle.

2. Composé suivant la revendication 1, ou la chaîne latérale $CH_2NHR^1$ est attachée à l'une des positions ci-après:

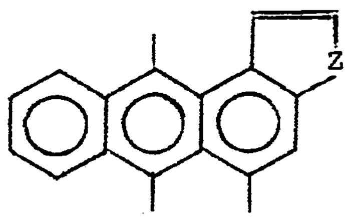

où Z est S, O, NMe ou NEt.

3. Composé suivant la revendication 1, qui est le 2-méthyl-2[[3-méthyl-3H-napht[2,3-e]-11-yl)méthyl]amino]-1,3-propanediol ou un sel d'addition d'acide de celui-ci.

4. Composé suivant la revendication 1, où $R^1$ est:

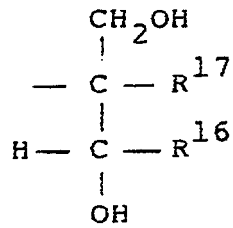

où $R^{16}$ est hydrogène ou méthyle et $R^{17}$ est hydrogène, méthyle ou éthyle.

5. Composition pharmaceutique comprenant un composé tel que défini dans l'une quelconque des revendications 1 à 4 et un excipient pharmaceutiquement acceptable.

6. Procédé pour préparer un composé de formule (I) tel que défini dans la revendication 1, lequel procédé comprend:

(a) la réduction d'un composé $ArCH{=}NR^1$ ou d'un dérivé convenablement protégé de celui-ci, suivie de la déprotection lorsqu'il y a lieu;

(b) la réduction d'un composé $Ar.CO.NHR^1$, où les radicaux hydroxyle sont éventuellement protégés, suivie de la déprotection des radicaux hydroxyle lorsqu'il y a lieu;

(c) la réaction d'un composé $ArCH_2L$, où L est un radical partant avec un composé $NH_2R^1$

7. Composé de formule (I) ou un éther, ester ou sel d'addition d'acide pharmaceutiquement acceptable de celui-ci, tel que défini dans la revendication 1 à utiliser dans le traitement de tumeurs.

8. Composé de formule (I) ou un éther, ester ou sel d'addition d'acide pharmaceutiquement acceptable de celui-ci, tel que défini dans la revendication 1 à utiliser dans la préparation d'un médicament pour le traitement de tumeurs.

**Revendications pour les Etats contractants: AT, ES, GR**

1. Procédé pour préparer un composé de formule (I):

$ArCH_2NHR^1$ (I)

ou un éther monométhylique ou monoéthylique de celui-ci; le composé de formule (I) y compris ces éthers ne comptant pas plus de 29 atomes de carbone au total; un ester de celui-ci; un sel de celui-ci;
où Ar est un système cyclique de formule:

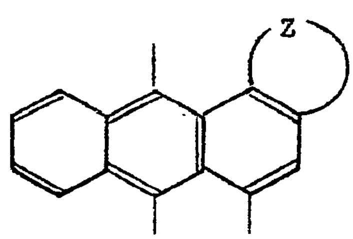

où la chaîne latérale $CH_2NHR^1$ est attachée à l'une des positions indiquées, où

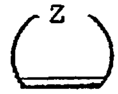

est un cycle de cinq chaînons contenant deux doubles liaisons et Z est oxygène, soufre ou azote substitué par hydrogène, méthyle ou éthyle, ou une forme de résonance de celui-ci,

Ar portant éventuellement un ou deux substituants, ces substituants ne comptant pas plus de quatre atomes de carbone au total lorsqu'ils sont pris ensemble et étant identiques et différents et choisis parmi halogène; cyano; $C_{1-4}$-alcoyle ou $C_{1-4}$-alcoxy, chacun éventuellement substitués par hydroxyle ou $C_{1-2}$-alcoxy; $C_{1-2}$-alcoyle ou $C_{1-2}$-alcoxy halogéno-substitué; un radical $S(O)_nR^2$, ou n est un entier 0, 1 ou 2 et $R^2$ est $C_{1-2}$-alcoyle éventuellement substitué par hydroxyle ou $C_{1-2}$-alcoxy; ou bien Ar est éventuellement substitué par un radical $NR^3R^4$ ne comptant pas plus de 5 atomes de carbone, où $R^3$ et $R^4$ sont identiques ou différents et sont chacun un radical $C_{1-3}$-alcoyle ou $NR^3R^4$ forme un hétérocycle de cinq ou six chaînons contenant éventuellement un ou deux hétéroatomes supplémentaires;
$R^1$ ne compte pas plus de huit atomes de carbone et est un radical:

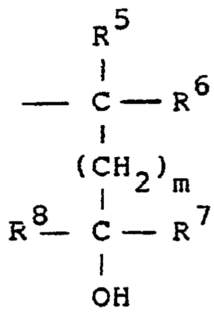

ou

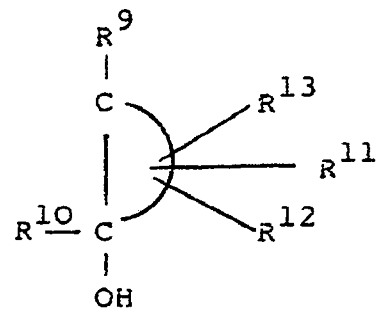

où est 0 ou 1; $R^5$ et $R^6$ sont identiques ou différents et sont chacun hydrogène ou $C_{1-3}$-alcoyle éventuellement substitué par hydroxyle; $R^7$ et $R^8$ sont identiques ou différents et sont chacun hydrogène ou $C_{1-3}$-alcoyle; –C–C est un carbocycle saturé de cinq ou six chaînons; $R^9$ est hydrogène, méthyle ou hydroxyméthyle; $R^{10}$, $R^{11}$ et $R^{12}$ sont identiques ou différents et sont chacun hydrogène ou méthyle; $R^{13}$ est hydrogène, méthyle, hydroxyle ou hydroxyméthyle, lequel procédé comprend:

(a) la réduction d'un composé $ArCH{=}NR^1$ ou d'un dérivé convenablement protégé de celui-ci, suivi de la déprotection lorsqu'il y a lieu;

(b) la réduction d'un composé $Ar.CO.NHR^1$, où les radicaux hydroxyle sont éventuellement protégés, suivie de la déprotection des radicaux hydroxyle lorsqu'il y a lieu;

(c) la réaction d'un composé $ArCH_2L$, où L est un radical partant avec un composé $NH_2R^1$

2. Procede suivant la revendication 1, pour préparer un composé, où la chaine latérale $CH_2NHR^1$ est attachée à l'une des positions ci-après:

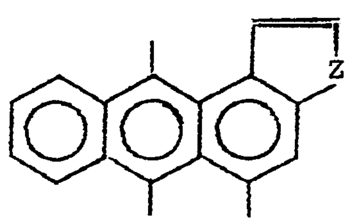

où Z est S, O, NMe ou NEt.

3. Procédé suivant la revendication 1, pour préparer un composé qui est le 2-méthyl-2-[[(3-méthyl-3H-naphth[2,3-e]indol-11-yl)méthyl]amino]-1,3-propanediol ou un sel d'addition d'acide de celui-ci.

4. Procédé suivant la revendication 1, pour préparer un composé où $R^1$ est:

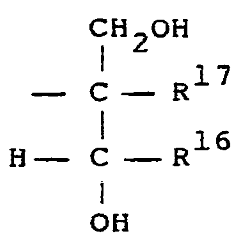

où $R^{16}$ est hydrogène ou méthyle et $R^{17}$ est hydrogène, méthyle ou éthyle.

5. Composition pharmaceutique comprenant un composé de formule (I) ou un éther ou sel d'addition d'acide pharmaceutiquement acceptable de celui-ci, tel que défini dans la revendication 1, et un excipient pharmaceutiquement acceptable.